(19) Europäisches Patentamt
European Patent Office
Office européen des brevets

(11) Veröffentlichungsnummer: **0 135 719**
**B1**

# (12) EUROPÄISCHE PATENTSCHRIFT

(45) Veröffentlichungstag der Patentschrift: **28.01.87**

(51) Int. Cl.⁴: **C 07 C 121/30**, C 07 C 121/70, C 07 C 120/00, A 61 K 7/46

(21) Anmeldenummer: **84109061.6**

(22) Anmeldetag: **01.08.84**

(54) **Alpha-tertiäre Nitrile, deren Herstellung und Verwendung als Duftstoffe.**

(30) Priorität: **05.08.83 DE 3328422**

(43) Veröffentlichungstag der Anmeldung: **03.04.85 Patentblatt 85/14**

(45) Bekanntmachung des Hinweises auf die Patenterteilung: **28.01.87 Patentblatt 87/5**

(84) Benannte Vertragsstaaten: **CH DE FR GB LI NL**

(56) Entgegenhaltungen:
**US - A - 3 655 722**

**TETRHEDRON LETTERS, Nr. 52, 1975. G. R. KIECZYKOWSKI et al.: "Regio-specific monoalkylation of 3-butenenitrile", Seiten 4647-4650**

(73) Patentinhaber: **Consortium für elektrochemische Industrie GmbH, Zielstattstrasse 20, D-8000 München 70 (DE)**

(72) Erfinder: **Gebauer, Helmut, Dr. Dipl.-Chem., Schaffhauser Strasse 18/VII, D-8000 München 71 (DE)**

**EP 0 135 719 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Nitrile, die, jeweils bezogen auf die Nitrilgruppe, ein tertiäres Kohlenstoffatom in α-Stellung, in β-γ-Position eine olefinische sowie in γ'-δ'-Position eine olefinische oder aromatische Struktureinheit aufweisen.

Es sind bereits Nitrile als Duftstoffe bekanntgeworden. Beispielsweise wird in US-PS 3 655 722 die Verwendung von 3.7-Dimethyl-2.6-octadiennitril als Riechstoff beschrieben. Daneben werden gemäss DE-OS 29 10 579 carbocyclische Nitrile als Duftstoffe vorgestellt.

Es ist ferner aus «Tetrahedron Letters», No. 52, 1975, 4647-4650 2-Ethenyl-2-methyl-4-penten--nitril und 2-Ethenyl-2-isopropyl-4-pentennitril bekannt, ohne dass in der genannten Veröffentlichung eine Verwendung angegeben wurde.

Aufgabe der Erfindung war es, Duftstoffe aufzufinden, die chemisch leicht und vollsynthetisch zugänglich sind.

Gegenstand der Erfindung sind Verbindungen der allgemeinen Formel

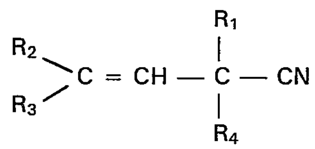

wobei
$R_1$ einen Alkylrest mit 1 bis 3 Kohlenstoffatomen darstellt,
$R_2$, $R_3$ Wasserstoff oder Alkylrest mit 1 bis 3 Kohlenstoffatomen bedeuten,
$R_4$ ein allylischer oder benzylischer Rest mit 3 bis 9 Kohlenstoffatomen oder ein Diphenylmethyl-Rest ist und wobei 2-Ethenyl-2-methyl-4-penten-nitril und 2-Ethenyl-2-isopropyl-4-pentennitril ausgeschlossen sind.

Beispiele für $R_1$ sind der Methyl-, der Ethyl-, der n-Propyl- und der iso-propyl-Rest.

Beispiele für $R_2$ und $R_3$ sind Wasserstoff und die gemäss $R_1$ gegebenen Beispiele.

$R_4$ ist ein allylischer oder benzylischer Rest mit 3 bis 9 Kohlenstoffatomen. Darunter werden solche Reste verstanden, die, bezogen auf die Nitrilgruppe, in γ'δ'-Stellung eine olefinische oder eine aromatische Struktureinheit aufweisen. Beispiel hierfür sind der Allyl-, der Methallyl-, der Crotyl-, der Prenyl-, der Benzyl-, der 4-Methoxybenzyl-, der 4-Methylbenzyl-, der 2.4-Dimethoxybenzyl- und der 2.4-Dimethylbenzyl-Rest. Ein weiteres Beispiel für $R_4$ ist der Diphenylmethyl-Rest.

Beispiele für erfindungsgemässe Verbindungen sind:

2-Ethenyl-2-ethyl-4-pentennitril
2-Ethenyl-2-n-propyl-4-pentennitril
2-Ethenyl-2-methyl-4-hexennitril
2-Ethenyl-2-ethyl-4-hexennitril
2-Ethenyl-2-n-propyl-4-hexennitril
2-Ethenyl-2-iso-propyl-4-hexennitril
2.5-Dimethyl-2-ethenyl-4-hexennitril
2-Ethyl-2-propenyl-4-hexennitril
2-Ethenyl-2-n-propyl-4-hexennirtil
2-Ethenyl-2-iso-propyl-4-hexennitril
2.4-Dimethyl-2-ethenyl-4-pentennitril
2-Ethenyl-2-ethyl-4-methyl-4-pentennitril
2-Ethenyl-2-n-propyl-4-methyl-4-pentennitril
2-Ethenyl-2-iso-propyl-4-methyl-4-pentennitril
2-Benzyl-2-methyl-3-butennitril
2-Benzyl-2-ethyl-3-butennitril
2-Benzyl-2-ethenyl-pentannitril
2-Benzyl-2-ethenyl-3-methyl-butannitril
2-(4-Methyl-phenylmethyl)-2-methyl-3-butennitril
2-(2.4-Dimethylphenylmethyl)-2-methyl-3-butennitril
2-(4-Methoxy-phenylmethyl)-2-methyl-3-butennitril
2-(2.4-Dimethoxy-phenylmethyl)-2-methyl-3--butennitril
2-Diphenylmethyl-2-methyl-3-butennitril
2-Propenyl-2-methyl-4-pentennitril
2-Butenyl-2-methyl-4-pentennitril
2-Pentenyl-2-methyl-4-pentennitril
2-Propenyl-2-ethyl-4-pentennitril
2-Propenyl-2-n-propyl-4-pentennitril
2-Propenyl-2-iso-propyl-4-pentennitril
2-Butenyl-2-ethyl-4-pentennitril
2-Butenyl-2-propyl-4-pentennitril
2-Butenyl-2-iso-propyl-4-pentennitril
2-Pentenyl-2-ethyl-4-pentennitril
2-Pentenyl-2-propyl-4-pentennitril
2-Pentenyl-2-iso-propyl-4-pentennitril
2-(2'-Methyl-propenyl)-2-methyl-4-pentennitril
2-(2-Methylbutenyl)-2-methyl-4-pentennitril
2-(2-Methylpentenyl)-2-methyl-4-pentennitril
2-(2-Ethyl-butenyl)-2-methyl-4-pentennitril
2-(2-Ethyl-pentenyl)-2-methyl-4-pentennitril
2-(2-Propyl-pentenyl)-2-methyl-4-pentennitril
2-(2-Methyl-propenyl)-2-ethyl-4-pentennitril
2-(2-Methyl-propenyl)-2-propyl-4-pentennitril
2-(2-Methyl-propenyl)-2-iso-propyl-4-pentennitril
2-(2.3-Dimethyl-butenyl)-2-methyl-4-pentennitril
2-Ethyl-2-ethenyl-5-methyl-4-hexennitril
2-Propyl-2-ethenyl-5-methyl-4-hexennitril
2-Propenyl-2.5-dimethyl-4-hexennitril
2-Butenyl-2.5-dimethyl-4-hexennitril
2-Pentenyl-2.5-dimethyl-4-hexennitril
2-(2-Methyl-propenyl)-2.5-dimethyl-4-hexennitril
2-(2-Methyl-pentenyl)-2.5-dimethyl-4-hexennitril
2-Propenyl-2-n-propyl-5-methyl-4-hexennitril
2-Propenyl-2-iso-propyl-5-methyl-4-hexennitril
2-Propenyl-2-ethyl-5-methyl-4-hexennitril
2-Butenyl-2-ethyl-5-methyl-4-hexennitril
2-Butenyl-2-n-propyl-5-methyl-4-hexennitril
2-Butenyl-2-iso-propyl-5-methyl-4-hexennitril
2-(2-Methyl-propenyl)-2-ethyl-5-methyl-4-hexennitril
2-(2-Methyl-propenyl)-2-n-propyl-5-methyl-4--hexennitril
2-(2-Methyl-butenyl)-2-ethyl-5-methyl-4-hexennitril
2-(2-Methyl-butenyl)-2-iso-propyl-5-methyl-4--hexennitril
2-(2-Methyl-butenyl)-2.5-dimethyl-4-hexennitril
2-Benzyl-2-propenyl-pentannitril
2-Ethenyl-2-methyl-4-heptennitril
2-Ethenyl-2-ethyl-4-heptennitril
2-Ethenyl-2-n-propyl-4-heptennitril

2-Ethenyl-2-iso-propyl-4-heptennitril
2-Propenyl-2-methyl-4-heptennitril
2-Propenyl-2-ethyl-4-heptennitril
2-Propenyl-2-n-propyl-4-heptennitril
2-Propenyl-2-iso-propyl-4-heptennitril
2-(4-Methoxy-phenylmethyl)-2-ethyl-3-butennitril.

Die erfindungsgemässen Verbindungen sind durch phasentransferkatalysierte Alkylierung α-β-ungesättigter Nitrile zugänglich.

Ein bevorzugtes Verfahren zur Herstellung der Verbindungen der allgemeinen Formel

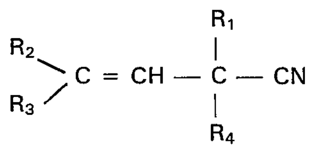

wobei
$R_1$ für einen Alkylrest mit 1 bis 3 Kohlenstoffatomen steht,
$R_2$, $R_3$ Wasserstoff oder Alkylrest mit 1 bis 3 Kohlenstoffatomen bedeutet und
$R_4$ ein allylischer oder benzylischer Rest mit 3 bis 9 Kohlenstoffatomen oder ein Diphenylmethyl-Rest ist,
das dadurch gekennzeichnet ist, dass Verbindungen der allgemeinen Formel

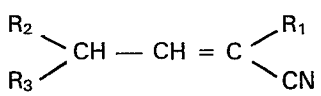

wobei
$R_1$, $R_2$ und $R_3$ die oben angegebene Bedeutung besitzen mit Verbindungen der Formel

$$R_4X$$

wobei
$R_4$ die oben angegebene Bedeutung besitzt und
X für Chlorid, Bromid oder Jodid steht,
in einem organisch/alkalischen 2-Phasen-System in Gegenwart eines Phasentransferkatalysators umgesetzt werden.

Beispiele für Verbindungen der allgemeinen Formel

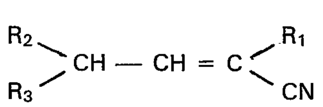

sind insbesondere 2-Methyl-2-butennitril, 2-Ethyl-2-butennitril, 2-Ethylidenpentannitril, 2-Methyl-2-pentennitril, 2-Ethyl-2-pentennitril, 2-Propyl-2-pentennitril, 2.4-Dimethyl-2-pentennitril, 2-Methyl-2-hexennitril, 2-Methyl-2-heptennitril und andere.

Die als Ausgangsverbindungen einzusetzenden Nitrile sind durch Cyanhydrinbildung an Ketonen und anschliessende Wasserabspaltung zugänglich. Die Herstellung entsprechender Ketone

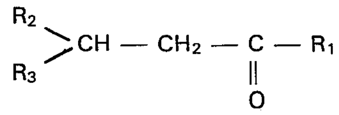

ist Stand der Technik.

Sie sind zum Teil handelsübliche Substanzen. Die Ketone sind beispielsweise als Aldolkondensationsprodukte oder vorteilhafterweise aus entsprechenden Carbonsäuregemischen darstellbar, die bei höheren Temperaturen über Tonerde- oder Thoriumoxidkontakte geleitet werden, wobei unter Kondensation und Decarboxylierung die entsprechenden Ketone gebildet werden.

Beispiele für Verbindungen der Formel $R_4X$, im folgenden auch als Alkylierungsmittel bezeichnet, sind Allylchlorid, Allylbromid, Methallylchlorid, Crotylchlorid, Prenylchlorid, Benzylchlorid, 4-Methylbenzylchlorid, 4-Methoxybenzylchlorid, 2.4-Dimethylbenzylchlorid, 2.4-Dimethoxybenzylchlorid, Diphenylmethylbromid und andere.

Das organisch/alkalische 2-Phasen-System wird gebildet aus einem organischen, mit Wasser nicht mischbaren, inerten Lösungsmittel und einer 5-50-%igen, insbesondere 20-50%igen wässrigen Lösung oder in fester Form vorliegendem Alkalimetallhydroxid.

Beispiele für inerte Lösungsmittel sind Benzol, Toluol, Xylol, Cyclohexan, Petrolether, Benzine und andere.

Beispiele für Alkalihydroxide sind insbesondere NaOH, KOH und andere.

Erfindungsgemäss können alle Phasentransferkatalysatoren eingesetzt werden, die auch bereits bisher zu derartigen Reaktionen verwendet werden konnten. Beispiele sind Kronenether, quartäre Ammonium- und Phosphoniumsalze, insbesondere Tetrabutylammoniumbromid. Die Phasentransferkatalysatoren werden in Mengen von 0,5 bis 5 Mol-%, insbesondere 2 bis 3 Mol-%, jeweils bezogen auf Alkylierungsmittel, eingesetzt.

Die Mengen an zu alkylierendem α-β-ungesättigtem Nitril und Alkylierungsmittel sind in etwa äquimolar. Oftmals wird jedoch ein Überschuss an Alkylierungsmittel eingesetzt, z.B. 1,1 bis 1,5 Mol Alkylierungsmittel pro Mol umzusetzenden Nitrils.

Bezogen auf die Menge des Alkylierungsmittels sind in etwa äquimolare Mengen an Alkalimetallhydroxid erforderlich. Vorteilhaft ist ein Überschuss von 1,1 bis 1,5 Mol Alkalimetallhydroxid pro Mol eingesetzten Alkylierungsmittels.

Die Reihenfolge der Zugabe der Reaktionskomponenten zum Reaktionsgemisch kann an sich beliebig gewählt werden. In einer vorteilhaften Ausführungsform des Verfahrens wird das organisch/alkalische 2-Phasen-System zusammen mit Phasentransferkatalysator vorgelegt und ein Gemisch der übrigen Reaktionskomponenten zudosiert.

Alternativ können ebenso alle organischen Reaktionskomponenten vorgelegt werden und Alkalimetallhydroxid, insbesondere in fester Form, zudosiert werden.

Die Reaktionstemperaturen werden im allgemeinen zwischen 0 und 150°C, insbesondere 20 bis 110°C gehalten. Oftmals wird ein optimales Verhältnis von Reaktionszeit und Ausbeute bei Temperaturen zwischen 60 und 90°C erzielt.

Die Aufarbeitung des Reaktionsgemisches kann durch konventionelle Technik erfolgen: gewöhnlich werden die Phasen getrennt und die organische Phase einer fraktionierten Destillation unterworfen.

Gegenstand der Erfindung ist weiterhin die Verwendung der erfindungsgemässen Verbindungen als Duftstoffe. Hierbei sind 2-Ethenyl-2-methyl-4-pentennitril und 2-Ethenyl-2-isopropyl-4-pentennitril nicht ausgeschlossen.

Mit den erfindungsgemässen Verbindungen sind in der Parfümerie neue, vorwiegend fruchtig-zitronige, aber auch blumig-grüne Duftnoten erzielbar. Zudem haftet manchen Produkten ein in Kompositionen gelegentlich erwünschter angenehmer Metallcharakter an.

Die Erfindung wird nun anhand von Beispielen näher erläutert:

*Beispiel 1*

2.5-Dimethyl-2-ethenyl-4-hexennitril

In einem 1-l-Vierhalskolben mit Rührer, Rückflusskühler und Tropftrichter wurden 48 g (1,2 Mol) festen Natriumhydroxids, 200 ml Toluol und 10 g Tetrabutylammoniumbromid vorgelegt und auf 70°C erwärmt. Unter Rühren wurde innerhalb von 2 Stunden ein Gemisch aus 81 g (1 Mol) 2-Methyl--2-butennitril und 125,4 g (1,2 Mol) Prenylchlorid zugetropft. Danach wurde die Reaktionsmischung noch 3 Stunden bei 75°C gerührt. Zur Aufarbeitung des Reaktionsgemisches wurde zunächst überschüssiges Natriumhydroxid und gebildetes Natriumchlorid mit Wasser ausgewaschen. Danach wurden die Phasen getrennt und die organische Phase mit Wasser neutralgewaschen. Nach Abziehen des Lösungsmittels wurde im Vakuum über eine 30-cm-Vigreux-Kolonne destilliert. Die Ausbeute an Zielprodukt betrug 62 g, entsprechend 41% der Theorie.

Farbloses Öl, Siedepunkt bei 12 Torr, 77°C.

Duftnote: stark, zitronig, fruchtig-süss, leicht metallisch.

*Beispiel 2*

2-Methyl-2-phenylmethyl-3-butennitril

In einem 500-ml-Vierhalskolben mit Rührer, Tropftrichter und Wasserabscheider wurden 126,5 g (1 Mol) Benzylchlorid, 81 g (1 Mol) 2-Methyl-2-butennitril, 100 ml Toluol und 10 g Tetrabutylammoniumbromid vorgelegt und auf Rückfluss erhitzt. Dieser Mischung wurden portionsweise 40 g (1 Mol) festen Natriumhydroxids zugegeben. Danach wurde das Reaktionsgemisch noch 1 Stunde unter Rückfluss gehalten. Danach wurde mit Wasser neutralgewaschen, die Phasen getrennt und die organische Phase analog Beispiel 1 destilliert. Die Ausbeute an Zielprodukt betrug 103,5 g, entsprechend 61% der Theorie.

Farbloses Öl, Siedepunkt bei 0,05 Torr, 76 bis 77°C.

Duftnote: langhaftender, zitronenartig-fruchtiger Duft mit blumiger Beinote.

*Beispiel 3*

2-(2-Propenyl)-2-propyl-3-pentennitril

In einem 250-ml-Vierhalskolben mit Rührer, Rückflusskühler und Tropftrichter wurden 20 g (0,5 Mol) festen Natriumhydroxids, 80 ml Toluol und 5 g Tetrabutylammoniumbromid vorgelegt und auf 80°C erwärmt. Diesem 2-Phasen-System wurde ein Gemisch aus 50 g (0,41 Mol) 2-Propyl-2-pentennitril und 31,4 g (0,41 Mol) Allylchlorid innerhalb von 30 Minuten zudosiert. Das Reaktionsgemisch wurde noch 2 Stunden auf einer Temperatur von 75°C gehalten. Danach wurde analog Beispiel 1 aufgearbeitet. Es wurde eine Ausbeute von 24 g, entsprechend 29,4% der Theorie an Zielprodukt erzielt.

Farbloses Öl, Siedebereich bei 12 Torr, 87 bis 90°C.

Duftnote: stark, fruchtig-zitronig, stengelig grün.

*Beispiel 4*

5-Methyl-2-(1-propenyl)-2-propyl-4-hexennitril

Es wurden analog Beispiel 1 64 g (1,6 Mol) NaOH, 200 ml Toluol und 15 g Tetrabutylammoniumbromid vorgelegt und auf 75°C erwärmt. Der Vorlage wurde ein Gemisch aus 147,6 g (1,2 Mol) 2-Propyl-2-pentennitril und 157 g (1,5 Mol) Prenylchlorid innerhalb von 2 Stunden zudosiert. Das Reaktionsgemisch wurde noch 3 Stunden bei 85°C gerührt und anschliessend analog Beispiel 1 aufgearbeitet. Die Ausbeute an Zielprodukt betrug 103 g, entsprechend 44,9% der Theorie.

Farbloses Öl, Siedebereich bei 0,05 Torr, 60 bis 65°C.

Duftnote: zitronig, fruchtig-süss, leicht fettig.

**Patentansprüche**

1. Verbindungen der allgemeinen Formel

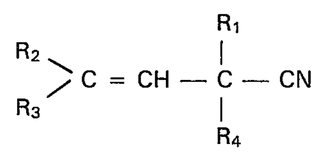

wobei

$R_1$ einen Alkylrest mit 1 bis 3 Kohlenstoffatomen darstellt,

$R_2$, $R_3$ Wasserstoff oder Alkylrest mit 1 bis 3 Kohlenstoffatomen bedeuten

$R_4$ ein allylischer oder benzylischer Rest mit 3 bis 9 Kohlenstoffatomen oder ein Diphenylmethyl-Rest ist und wobei 2-Ethenyl-2-methyl-4-pentennitril und 2-Ethenyl-2-isopropyl-4-pentennitril ausgeschlossen sind.

2. Verfahren zur Herstellung von Verbindungen nach Anspruch 1, dadurch gekennzeichnet, dass Verbindungen der allgemeinen Formel

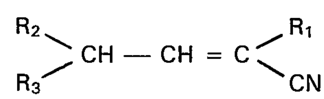

wobei

$R_1$, $R_2$ und $R_3$ die gemäss Anspruch 1 gegebene Bedeutung besitzen
mit Verbindungen der Formel

$R_4X$

wobei
$R_4$ die gemäss Anspruch 1 gegebene Bedeutung besitzt und
X für Chlorid, Bromid oder Jodid steht,
in einem organisch/alkalischen 2-Phasen-System in Gegenwart eines Phasentransferkatalysators umgesetzt werden.

3. Verwendung von Verbindungen nach Anspruch 1, 2-Ethenyl-2-methyl-4-pentennitril und 2-Ethenyl-2-isopropyl-4-pentennitril als Duftstoffe.

**Claims**

1. Compounds of the general formula

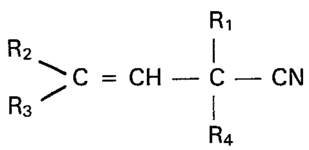

wherein
$R_1$ represents an alkyl radical having from 1 to 3 carbon atoms,
$R_2$, $R_3$ represent hydrogen or alkyl radical having from 1 to 3 carbon atoms,
$R_4$ is an allylic or benzylic radical having from 3 to 9 carbon atoms or a diphenylmethyl radical, 2-ethenyl-2-methyl-4-pentenenitrile and 2-ethenyl-2-isopropyl-4-pentenenitrile being excluded.

2. Process for the manufacture of compounds according to claim 1, characterised in that compounds of the general formula

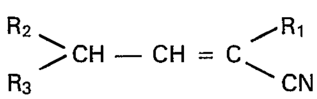

wherein
$R_1$, $R_2$ and $R_3$ have the meanings given in claim 1, are reacted with compounds of the formula

$R_4X$

wherein
$R_4$ has the meaning given in claim 1 and
X represents chloride, bromide or iodide,
in an organic/alkaline two-phase system in the presence of a phase transfer catalyst.

3. Use of compounds according to claim 1, 2-ethenyl-2-methyl-4-pentenenitrile and 2-ethenyl-2-isopropyl-4-pentenenitrile as fragrant substances.

**Revendications**

1. Composés répondant à la formule générale suivante:

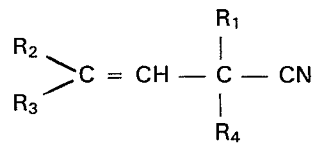

dans laquelle
$R_1$ représente un radical alkyle contenant de 1 à 3 atomes de carbone,
$R_2$ et $R_3$ représentent chacun l'hydrogène ou un radical alkyle contenant de 1 à 3 atomes de carbone et
$R_4$ représente un radical allylique ou benzylique contenant de 3 à 9 atomes de carbone ou un radical diphénylméthyle, sauf l'éthényl-2 méthyl-2 pentène-4 nitrile et l'éthényl-2 isopropyl-2 pentène-4 nitrile.

2. Procédé de préparation de composés selon la revendication 1, procédé caractérisé en ce qu'on fait réagir des composés répondant à la formule générale:

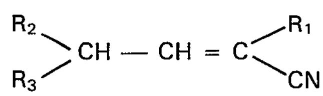

dans laquelle
$R_1$, $R_2$ et $R_3$ ont les significations données à la revendication 1, avec des composés répondant à la formule:

$R_4X$

dans laquelle
$R_4$ a la signification donnée à la revendication 1 et
X représente le chlore, le brome ou l'iode,
dans un système à 2 phases organique/alcalin, en présence d'un catalyseur de transfert de phase.

3. Application de composés selon la revendication 1, de l'éthényl-2 méthyl-2 pentène-4 nitrile et de l'éthényl-2 isopropyl-2 pentène-4 nitrile comme parfums.